# EUROPEAN PATENT APPLICATION

(11) **EP 3 598 969 A1**
(43) Date of publication of application: **29.01.2020**
(21) Application number: 19188261.2
(22) Date of filing: 25.07.2019
(51) Int. Cl.: A61K 31/05, A61K 31/352, A61K 36/185, B02C 23/38, B02C 23/18

(54) **METHOD AND APPARATUS FOR HARVESTING TRICHOMES FROM CANNABIS PLANTS**

(30) Priority: 26.07.2018 US 201862703589 P
(71) Applicant: AIR PRODUCTS AND CHEMICALS, INC., Allentown, Pennsylvania 18195 (US)
(72) Inventor: HIMES, Michael Robert, Macungie, PA Pennsylvania 18062 (US); BENDER, Eric Charles, Hamburg, PA Pennsylvania 19526 (US); LANHAM, Mark Wayne, Defiance, MO Missouri 63341 (US); SHLANTA, Andrew Vincent, New Ringgold, PA Pennsylvania 17960 (US)
(74) Representative: Kador & Partner PartG mbB

(57) **Abstract**

A method of harvesting trichomes from a cannabis plant piece containing one or more trichomes attached to biomass material, the biomass material including one or more of stalk material, flower material, and leaf material, including freezing a cannabis plant piece by exposing the plant piece to a cryogenic fluid to create a frozen plant piece; processing the frozen plant piece to separate the trichomes from the biomass material using one or more screens; removing the biomass that has been prevented from passing through the one or more screens; and collecting the trichomes that have passed through the one or more screens.

## Description

### CROSS REFERENCED TO RELATED APPLICATIONS

This application claims the priority of US Provisional Patent Application No. 62/703,589 filed July 26, 2018, which is incorporated by reference herein in its entirety.

### BACKGROUND

The present invention relates to an apparatus and method of harvesting trichomes from plants in the cannabis family, including but not limited to industrial hemp plants, for at least the purpose of extracting cannabidiol (CBD) oil.

In order to obtain CBD oil from cannabis plants, the current practice is to dry the plant and chop it into smaller pieces so that the oil can be chemically extracted. The oil is contained in trichomes located on the flowers of the cannabis plant. When the plant is chopped, the trichomes can smear or the heat generated during the chopping process can vaporize the oil thus decreasing the amount of oil recovered.

### SUMMARY

One benefit of the methods and devices described herein is to reduce the amount of non-trichome-containing material introduced into the extraction process, thereby making the downstream extraction process more efficient and decreasing the amount of extraction fluid required.
Aspect 1. A method of harvesting trichomes from a cannabis plant piece containing one or more trichomes attached to biomass material, the biomass material including one or more of stalk material, flower material, and leaf material, comprising: freezing a cannabis plant piece by exposing the plant piece to a cryogenic fluid to create a frozen plant piece; processing the frozen plant piece to separate the trichomes from the biomass material using one or more screens; removing the biomass that has been prevented from passing through the one or more screens; and collecting the trichomes that have passed through the one or more screens.
Aspect 2. The method of Aspect 1, where the processing step comprises: subjecting the frozen plant piece to impact to detach the trichomes from the biomass material and to vibration on a screen to separate the trichomes, which are sufficiently small to pass through the screen, from the biomass, which is sufficiently large to be prevented from passing through the screen.
Aspect 3. The method of Aspect 1 or Aspect 2, wherein the processing step occurs while continuing to expose the frozen plant piece to the cryogenic fluid to maintain it in a frozen state.
Aspect 4. The method of any one of Aspects 1 to 3, wherein the frozen plant piece is maintained in a frozen state at less than or equal to -45 °C.
Aspect 5. The method of claim 1, wherein the processing step comprises: while continuing to expose the frozen plant piece to the cryogenic fluid to maintain it in a frozen state: subjecting the frozen plant piece to impact to detach the trichomes from the biomass material and to vibration on a first screen to separate the trichomes and flower material and leaf material, which are sufficiently small to pass through the first screen, from the stalk material, which is sufficiently large to be prevented from passing through the first screen; and subjecting the trichomes and flower material and leaf material to vibration on a second screen having a mesh size smaller than the first screen to separate the trichomes, which are sufficiently small to pass through the first screen, from the flower material and leaf material, which are sufficiently large to be prevented from passing through the second screen.
Aspect 6. A device for harvesting trichomes from a cannabis plant piece containing one or more trichomes attached to biomass material, the biomass material including one or more of stalk material, flower material, and leaf material, comprising: an upper chamber having a top configured and arranged to receive frozen cannabis plant pieces, a bottom containing a first screen having a mesh size sufficiently large to allow passage through of trichomes but sufficiently small to prevent passage through of at least a portion of the biomass material, and a sidewall having an outlet for discharge of the portion of the biomass material prevented from passing through the screen; and impactors positioned so as to move freely within the upper chamber, such that when the upper chamber is vibrated the impactors collide with the frozen cannabis plant pieces to dislodge trichomes from the biomass material and the trichomes that have been dislodged pass through the first screen.
Aspect 7. The device of Aspect 6, further comprising: a collection pan positioned beneath the upper chamber and being configured and arranged to receive and collect the trichomes passed through the first screen.
Aspect 8. The device of any one of Aspect 6 or 7, further comprising: a vibrator positioned beneath the upper chamber configured and arranged to deliver vibration to the upper chamber to cause the impactors to collide with the frozen cannabis plant pieces and to cause separation of the trichomes from the biomass material in the upper chamber and to cause the trichomes to pass through the first screen.
Aspect 9. The device of Aspect 6, further comprising: a hopper configured and arranged to receive one or more frozen cannabis plant pieces and to deliver the frozen cannabis plant pieces into the top of the upper chamber.
Aspect 10. The device of Aspect 9, further comprising: a spray header configured and arranged to delivery cryogenic fluid into the hopper to maintain the cannabis plant pieces in a frozen state.
Aspect 11. The device of any one of Aspects 6 to 10, wherein the first screen has a mesh size that allows passage through of flower material and leaf material but prevents passage through of stalk material, further comprising: a lower chamber positioned beneath the upper chamber and having a top configured to receive the trichomes, flower material, and leaf material passed through the first screen, a bottom containing a second screen having a mesh size sufficient to allow passage through of trichomes but to prevent passage through of flower material and leaf material, and a sidewall having an outlet for discharge of flower material and leaf material.
Aspect 12. The device of Aspect 11, further comprising: a collection pan positioned beneath the lower chamber and being configured and arranged to receive and collect the trichomes passed through the second screen.
Aspect 13. The device of Aspect 11 or Aspect 12, further comprising: a vibrator positioned beneath the lower chamber configured and arranged to deliver vibration to the upper chamber and the lower chamber to cause the impactors to collide with the cannabis plant pieces in the upper chamber and to cause separation of the trichomes, flower material, and leaf material from the stalk material in the upper chamber, to cause the trichomes, the flower material, and the leaf material to pass through the first screen, and to the trichomes to pass through the second screen.
Aspect 14. The device of any one of Aspects 11 to 13, further comprising a temperature sensor positioned in the lower chamber to enable control of a flow of cryogenic fluid through the spray header such that the temperature of the lower chamber is maintained at less than or equal to -45 °C.
Aspect 15. The device of any one of Aspects 11 to 14, wherein the first screen is 6-mesh or smaller, and wherein the second screen has a mesh size smaller than that of the first screen.
Aspect 16. A device for harvesting trichomes from a cannabis plant piece containing one or more trichomes attached to biomass material, the biomass material including one or more of stalk material, flower material, and leaf material, comprising: a hopper configured and arranged to receive one or more frozen cannabis plant pieces; a spray header configured and arranged to delivery cryogenic fluid into the hopper to maintain the cannabis plant pieces in a frozen state; an upper chamber having a top configured and arranged to receive frozen cannabis plant pieces from the hopper, a bottom containing a first screen having a mesh size sufficiently large to allow passage through of trichomes, flower material, and leaf material, but sufficiently small to prevent passage through of stalk material, and a sidewall having an outlet for discharge of the stalk material prevented from passing through the screen; impactors positioned so as to move freely within the upper chamber, such that when the upper chamber is vibrated the impactors collide with the frozen cannabis plant pieces to dislodge trichomes from the biomass material and to break apart the biomass material into flower material, leaf material, and stalk material, and the trichomes, the flower material, and the leaf material that have been dislodged pass through the first screen; a lower chamber positioned beneath the upper chamber and having a top configured to receive the trichomes, flower material, and leaf material passed through the first screen, a bottom containing a second screen having a mesh size sufficient to allow passage through of trichomes but to prevent passage through of flower material and leaf material, and a sidewall having an outlet for discharge of flower material and leaf material; a collection pan positioned beneath the lower chamber and being configured and arranged to receive and collect the trichomes passed through the second screen; and a vibrator positioned beneath the collection pan configured and arranged to deliver vibration to the upper chamber and the lower chamber to cause the impactors to collide with the cannabis plant pieces in the upper chamber and to cause separation of the trichomes, flower material, and leaf material from the stalk material in the upper chamber and separation of the trichomes from the flower material and the leaf material in the lower chamber.

### BRIEF DESCRIPTION OF THE DRAWINGS

The present invention will hereinafter be described in conjunction with the appended figures wherein like numerals denote like elements:
Fig. 1 is a schematic view of an embodiment of a method of harvesting trichomes from cannabis plants using cryogenic freezing.
Fig. 2 is a schematic view of an embodiment of another method of harvesting trichomes from cannabis plants using cryogenic freezing.

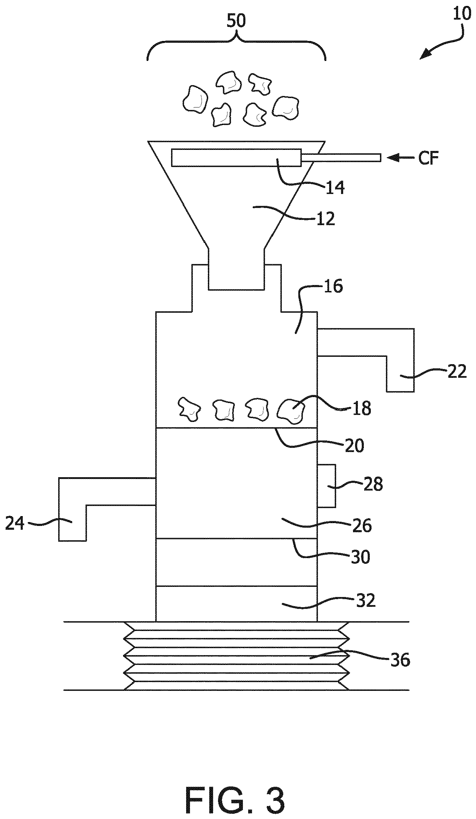

Fig 3 is a schematic of a screening device for separating the trichomes from cannabis plants using cryogenic freezing.

### DETAILED DESCRIPTION

A method and apparatus are described herein for freezing the trichomes from cannabis plants using cryogenic liquid or gas in order to prevent smearing and vaporizing CBD oil, and for subsequently separating the trichomes from the plants.

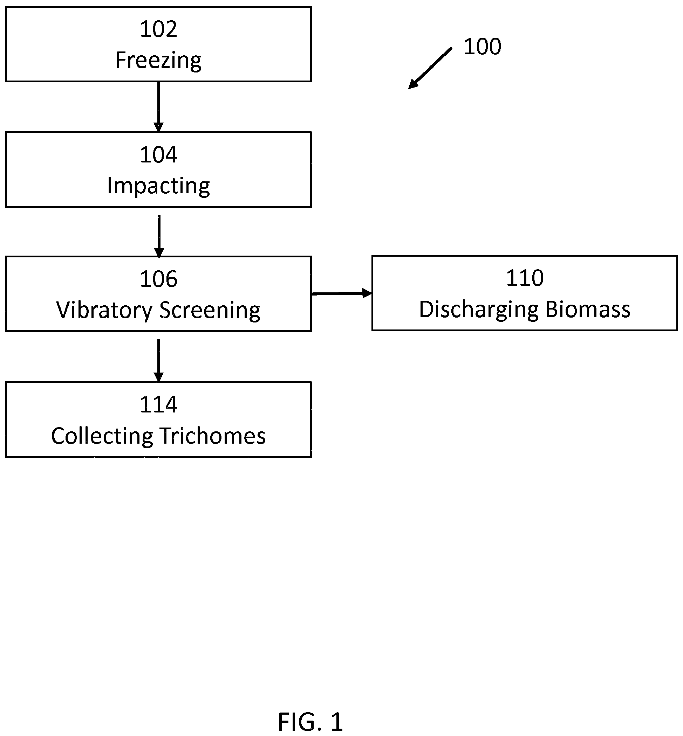

Fig. 1 shows an embodiment of a method or process 100 to harvest the trichomes from a cannabis plant including a Step 102 of Freezing, a Step 104 of Impacting, a Step 106 of Vibratory Screening, a Step 110 of Discharging Biomass, and a Step 114 of Collecting Trichomes.

In the Step 102, pieces of a cannabis plant are frozen by exposure to a cryogenic fluid, which may be a sprayed cryogenic liquid or a cryogenic gas. Preferably the cannabis plant, or pieces of the cannabis plant, are frozen directly after harvesting or after drying.

Next, in the Step 104, the frozen cannabis plant pieces are subjected to impacting to reduce the size of the plant. This can be done using a mill, a chopper, and/or a piece of equipment to strip off the leaves and flowers. In one embodiment, the impacting is accomplished by placing the frozen cannabis plant pieces in a chamber with impactors or hard objects, such as metal balls, rubber stoppers, or the like, that repeatedly subject the plant pieces to impacts or high forces as the chamber is vibrated or shaken. The impacts break apart the plant pieces and separate the trichomes from other parts of the plant such as flower material, leaf material, and stalk material. The flower material, leaf material, and stalk material are collectively referred to as biomass material. Generally, the stalk material remains the largest in size, followed by the flower material and plant material, and the trichomes are the smallest in size after separation from the biomass material.

Next, in Step 106, the sized-reduced and separated trichomes and biomass material are subjected to vibrations over or on a screen having a mesh sufficiently small to prevent passage of most or all of the biomass material but sufficiently large to allow passage through of the trichomes. Thus, the trichomes are separated from the biomass material.

It should be noted that during Steps 102, 104, and 106, the plant pieces, trichomes, and biomass material are maintained in a frozen state by continued addition of cryogenic fluid into the process. In some embodiments, the temperature of the plant pieces and/or the trichomes and biomass material may be controlled to at or below a predetermined temperature, such as -45 °C, to maintain both the desired mechanical properties that allow for good separation during the impacting and vibratory screening steps as well as preserve the quality of the trichomes.

In Step 110, the biomass material is discharged, while in Step 114 the trichomes are collected after passing through the screen.

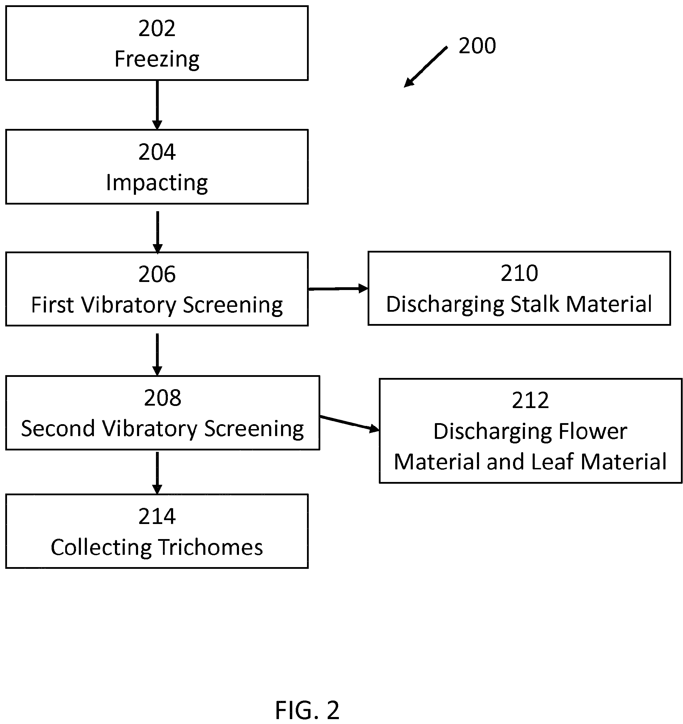

Fig. 2 shows another embodiment of a method or process 200 to harvest the trichomes from a cannabis plant including a Step 202 of Freezing, a Step 204 of Impacting, a Step 206 of First Vibratory Screening, a Step 208 of Second Vibratory Screening, a Step 210 of Discharging Biomass, and a Step 214 of Collecting Trichomes.

In the Step 202, pieces of a cannabis plant are frozen by exposure to a cryogenic fluid, which may be a sprayed cryogenic liquid or a cryogenic gas. Preferably the cannabis plant is frozen directly after harvesting or after drying.

Next, in the Step 204, the frozen cannabis plant pieces are subjected to impacting to reduce the size of the plant. This can be done using a mill, a chopper, and/or a piece of equipment to strip off the leaves and flowers. In one embodiment, the impacting is accomplished by placing the frozen cannabis plant pieces in a chamber with impactors or hard objects, such as metal balls, rubber stoppers, or the like, that repeatedly subject the plant pieces to impacts or high forces as the chamber is vibrated or shaken. The impacts break apart the plant pieces and separate the trichomes from other parts of the plant such as flower material, leaf material, and stalk material. The flower material, leaf material, and stalk material are collectively referred to as biomass material. Generally, the stalk material remains the largest in size, followed by the flower material and plant material, and the trichomes are the smallest in size after separation from the biomass material.

Next, in Step 206, the sized-reduced and separated trichomes and biomass material are subjected to vibrations over or on a first screen having a mesh sufficiently small to prevent passage of most or all of the stalk material but sufficiently large to allow passage through of the trichomes as well as most or all of the flower material and leaf material.

Next, in Step 208, the trichomes and the flower material and leaf material that passed through the first screen are subjected to vibrations over on or on a second screen having a mesh sufficiently small to prevent passage of most or all of the flower material and leaf material, but sufficiently large to allow passage through of the trichomes. Thus, the trichomes are separated from the biomass material. A two-stage separation tends to improve the recovery of trichomes and to maximize the removal of biomass material from the collected trichomes as compared with a single-stage separation.

It should be noted that during Steps 202, 204, 206, and 208, the plant pieces, trichomes, and biomass material are maintained in a frozen state by continued addition of cryogenic fluid into the process. In some embodiments, the temperature of the plant pieces and/or the trichomes and biomass material may be controlled to at or below a predetermined temperature, such as -45 °C, to maintain both the desired mechanical properties that allow for good separation during the impacting and vibratory screening steps as well as preserve the quality of the trichomes.

In Step 210, the stalk material is discharged after the first vibratory screening, in Step 212, the flower material and the leaf material are discharged after the second vibratory screening, while in Step 214, the trichomes are collected after passing through the second screen.

The plant may be frozen as a batch or continuous process using cryogenic liquid or gas, which may include, but is not limited to, liquid nitrogen, cold gaseous nitrogen, carbon dioxide snow, or cold carbon gaseous carbon dioxide.

For continuous freezing, the plants or pieces of plants are loaded on conveyor belts or conveyed on hooks through the freezing zone, for example in a tunnel freezer or the like. Immediately after the plant is frozen the plant is size reduced and then subjected to vibration using a vibrator screen. The vibrations and impacts separate the trichomes from the flowers. Cryogenic liquid or gas can be introduced to keep the plant and trichomes frozen during the vibration and size reduction process.

Fig. 2 shows an example of a screening device 10 used to separate the trichomes from the flower material. The frozen plant material 50, which has already been chopped into pieces, is fed into a hopper 12. Cryogenic liquid or gas CF is introduced in the hopper 12 via a manifold or spray header 14 in order to keep plant material frozen while being processed. The plant material descends by gravity and enters an upper chamber 16 and is subjected to high forces from the vibration of impactors 18, such as metal balls, rubber stoppers, or similar hard articles. The vibration of the upper chamber 16 is driven by a vibrator 36 positioned beneath the device 10. The leaves, flowers, and trichomes pass through a screen 20 of 6-mesh or smaller, while the stalk material remains in the upper chamber 16. The stalk material can discharge through a port 22 that is elevated above the screen 20 or is blocked by a gate (not shown) which is opened periodically to allow stalk material which does not contain trichomes to exit the upper chamber 16.

The leaves, flowers, and trichomes enter a lower chamber 26 and are further subjected to vibrations in order to separate the trichomes from the flowers and leaves (biomass). The lower chamber 26 is also vibrated by the vibrator 36. The trichomes pass through a screen 30 of a mesh smaller than that of the screen 20 into a collection pan 32. A discharge chute 24 is positioned above the screen 30 to collect the biomass material. A temperature sensor 28 is positioned in the lower chamber 26 to enable control of the amount of cryogen introduced into the hopper 12 such that the lower chamber 26 is maintained at a temperature less than or equal to - 45 °C (-50 °F). The first and second chambers are vibrated with a vibrator 26.

While the principles of the invention have been described above in connection with preferred embodiments, it is to be clearly understood that this description is made only by way of example and not as a limitation of the scope of the invention.

## Claims

1. A method of harvesting trichomes from a cannabis plant piece containing one or more trichomes attached to biomass material, the biomass material including one or more of stalk material, flower material, and leaf material, comprising:
freezing a cannabis plant piece by exposing the plant piece to a cryogenic fluid to create a frozen plant piece;
processing the frozen plant piece to separate the trichomes from the biomass material using one or more screens;
removing the biomass that has been prevented from passing through the one or more screens; and
collecting the trichomes that have passed through the one or more screens.

2. The method of claim 1, where the processing step comprises:
subjecting the frozen plant piece to impact to detach the trichomes from the biomass material and to vibration on a screen to separate the trichomes, which are sufficiently small to pass through the screen, from the biomass, which is sufficiently large to be prevented from passing through the screen.

3. The method of claim 2, wherein the processing step occurs while continuing to expose the frozen plant piece to the cryogenic fluid to maintain it in a frozen state.

4. The method of claim 3, wherein the frozen plant piece is maintained in a frozen state at less than or equal to -45 °C.

5. The method of any one of the preceding claims, wherein the processing step comprises:
while continuing to expose the frozen plant piece to the cryogenic fluid to maintain it in a frozen state:
subjecting the frozen plant piece to impact to detach the trichomes from the biomass material and to vibration on a first screen to separate the trichomes and flower material and leaf material, which are sufficiently small to pass through the first screen, from the stalk material, which is sufficiently large to be prevented from passing through the first screen; and
subjecting the trichomes and flower material and leaf material to vibration on a second screen having a mesh size smaller than the first screen to separate the trichomes, which are sufficiently small to pass through the first screen, from the flower material and leaf material, which are sufficiently large to be prevented from passing through the second screen.

6. A device for harvesting trichomes from a cannabis plant piece containing one or more trichomes attached to biomass material, the biomass material including one or more of stalk material, flower material, and leaf material, comprising:
an upper chamber having a top configured and arranged to receive frozen cannabis plant pieces, a bottom containing a first screen having a mesh size sufficiently large to allow passage through of trichomes but sufficiently small to prevent passage through of at least a portion of the biomass material, and a sidewall having an outlet for discharge of the portion of the biomass material prevented from passing through the screen; and
impactors positioned so as to move freely within the upper chamber, such that when the upper chamber is vibrated the impactors collide with the frozen cannabis plant pieces to dislodge trichomes from the biomass material and the trichomes that have been dislodged pass through the first screen.

7. The device of claim 6, further comprising:
a collection pan positioned beneath the upper chamber and being configured and arranged to receive and collect the trichomes passed through the first screen.

8. The device of claim 6 or 7, further comprising:
a vibrator positioned beneath the upper chamber configured and arranged to deliver vibration to the upper chamber to cause the impactors to collide with the frozen cannabis plant pieces and to cause separation of the trichomes from the biomass material in the upper chamber and to cause the trichomes to pass through the first screen.

9. The device of any one of claims 6 to 8, further comprising:
a hopper configured and arranged to receive one or more frozen cannabis plant pieces and to deliver the frozen cannabis plant pieces into the top of the upper chamber.

10. The device of claim 9, further comprising:
a spray header configured and arranged to delivery cryogenic fluid into the hopper to maintain the cannabis plant pieces in a frozen state.

11. The device of any one of claims 6 to 10, wherein the first screen has a mesh size that allows passage through of flower material and leaf material but prevents passage through of stalk material, further comprising:
a lower chamber positioned beneath the upper chamber and having a top configured to receive the trichomes, flower material, and leaf material passed through the first screen, a bottom containing a second screen having a mesh size sufficient to allow passage through of trichomes but to prevent passage through of flower material and leaf material, and a sidewall having an outlet for discharge of flower material and leaf material.

12. The device of claim 11, further comprising:
a collection pan positioned beneath the lower chamber and being configured and arranged to receive and collect the trichomes passed through the second screen.

13. The device of claim 11 or 12, further comprising:
a vibrator positioned beneath the lower chamber configured and arranged to deliver vibration to the upper chamber and the lower chamber to cause the impactors to collide with the cannabis plant pieces in the upper chamber and to cause separation of the trichomes, flower material, and leaf material from the stalk material in the upper chamber, to cause the trichomes, the flower material, and the leaf material to pass through the first screen, and to the trichomes to pass through the second screen.

14. The device of any one of claims 11 to 13, further comprising a temperature sensor positioned in the lower chamber to enable control of a flow of cryogenic fluid through the spray header such that the temperature of the lower chamber is maintained at less than or equal to - 45 °C.

15. The device of any one of claims 11 to 14, wherein the first screen is 6-mesh or smaller, and wherein the second screen has a mesh size smaller than that of the first screen.
